# EUROPÄISCHE PATENTSCHRIFT

(11) **EP 1 392 673 B1**
(45) Veröffentlichungstag und Bekanntmachung des Hinweises auf die Patenterteilung: **08.06.2005**
(21) Anmeldenummer: 02711818.1
(22) Anmeldetag: 28.01.2002
(51) Int. Cl.: C07D 333/16, C07D 213/30, C07D 239/26, C07D 333/36, C07D 333/28, C07D 213/89, C07D 277/20, A61K 7/13

(54) **1,3-DIHYDROXYBENZOL-DERIVATE UND DIESE VERBINDUNGEN ENTHALTENDE FÄRBEMITTEL**
1,3-DIHYDROXYBENZENE DERIVATIVES AND COLORANTS CONTAINING SAID COMPOUNDS
DERIVES DE 1,3-DIHYDROXYBENZENE ET COLORANTS CONTENANT CES COMPOSES

(30) Priorität: 25.05.2001 DE 10125453; 25.05.2001 DE 20108704 U
(43) Veröffentlichungstag der Anmeldung: 03.03.2004
(73) Patentinhaber: Wella Aktiengesellschaft, 64274 Darmstadt (DE)
(72) Erfinder: CHASSOT, Laurent, CH-1724 Praroman (CH); BRAUN, Hans-Jürgen, CH-3182 Überstorf (CH)
(74) Vertreter: Bockhorst, Matthias
(86) Internationale Anmeldenummer: PCT/EP2002/000850
(87) Internationale Veröffentlichungsnummer: WO 2002/096901

(56) Entgegenhaltungen:
- EP-A- 0 904 774
- DE-A- 3 233 541
- DE-A- 19 859 800
- DE-U- 20 110 355
- DE-U- 20 110 356
- FR-A- 1 396 684
- US-A- 3 893 803
- ATTIA ET AL: "Chemical and biological reactivity of newly synthesized 2-chloro-6-ethoxy-4-acetylpyridine" EGYPTION JOURNAL OF CHEMISTRY, Bd. 38, Nr. 5, 1995, Seiten 543-553, XP002212378
- PATER R. ET AL: "photostable o-hydroxyphenylquinazolines" JOURNAL OF HETEROCYCLIC CHEMSTRY, Bd. 7, Nr. 5, 1970, Seiten 1113-1124, XP002212379
- SARTORI G. ET AL: "Selective Synthesis of Unsymmetrical Hydrxylated and methoxylated biaryls" JOURNAL OF ORGANIC CHEMISTRY, Bd. 58, 1993, Seiten 7271-7273, XP002212380 USA
- KARPEL VEL LEITNER N. ET AL: "Kinetics and mechanics of the photolytic and OH radical induced oxidation of fluorinated aromatic compounds in aqueous solutions" CHEMOSPHERE, Bd. 32, Nr. 5, 1996, Seiten 893-906, XP002212381
- HOGBERG H-E ET AL: "DIBENZOFURAUS FROM THE ACID-CATALYSED REACTION OF ALKYL-P-BENZOQUINONES WITH RECORCINOL" ACTA CHEMICA SCANDINAVICA. SERIES B - ORGANIC CHEMISTRY AND BIOCHEMISTRY, MUNKSGAARD. COPENHAGEN, DK, Nr. 33, 1979, Seiten 271-276, XP001068463
- DOL G. ET AL: "Synthesis of 5-substituted resorcinol derivatives via cross-coupling reactions" EUROPEAN JOURNAL OF ORGANIC CHEMISTRY, 1998, Seiten 359-364, XP002212382 Weinheim
- PUMMERER R ET AL: "DIE KONDENSATION VON CHINONEN MIT PHENOLEN. (3. MITTEILUNG UEBER DIARYLCHINONE" CHEMISCHE BERICHTE, XX, XX, Nr. 60, 1927, Seiten 1442-1451, XP001068313 ISSN: 0009-2940
- CHANDRASEKHARAN V ET AL: "SYNTHESIS OF 5-ALKYL- & 5-ARYL-RESORCINOLS" INDIAN JOURNAL OF CHEMISTRY, JODHPUR, IN, Nr. 16B, November 1978 (1978-11), Seiten 970-972, XP001068323
- RAISTRICK H. ET AL: "Studies in the biochemistry of micro-organisms" THE BIOCHEMICAL JOURNAL, Bd. 55, 1953, Seiten 421-433, XP002212383
- ERDTMAN H ET AL: "the synthesis of unsymmetrical biphenyl derivatives by mixed ullmann coupling" ACTA CHEMICA SCANDINAVICA, Bd. 15, 1961, Seiten 1761-1764, XP002212384
- NILSSON M ET AL: "syntheses of aucuparin and methoxyaucuparin" ACTA CHEMICA SCANDINAVICA, Bd. 17, 1963, Seiten 1157-1159, XP002212385
- DEVLIN J.P.: "6H-Dibenzo[b,d]pyrans I Synthesis" CANADIAN JOURNAL OF CHEMISTRY, Bd. 53, 1975, Seiten 343-349, XP002212386
- SPATH E. ET AL: "Über die Konstitution des Sappanins" MONATSHEFTE DER CHEMIE, 1930, Seiten 342-351, XP002212387
- LÜNING U ET A L: "Concave reagents-14. Concave pyridines with a 2,6-diarylpyridine core" TETRAHEDRON LETTERS, Bd. 34, Nr. 32, Seiten 5063-5066, XP002212746 GB
- ASTLES PETER C ET AL: "Selective Endothelin A Receptor Antagonists. 3. Discovery and Structure-Activity Relationships of a Series of 4-Phenoxybutanoic Acid Derivatives" JOURNAL OF MEDICINAL CHEMISTRY, AMERICAN CHEMICAL SOCIETY. WASHINGTON, US, Bd. 41, 1998, Seiten 2732-2744, XP002177959 ISSN: 0022-2623
- JAXA-CHAMIEC A A ET AL: "A NEW ROUTE TO 5-SUBSTITUTED RESORCINOLS AND RELATED SYSTEMS" JOURNAL OF THE CHEMICAL SOCIETY, PERKIN TRANSACTIONS 1, CHEMICAL SOCIETY. LETCHWORTH, GB, 1980, Seiten 170-175, XP001069142 ISSN: 0300-922X

## Beschreibung

Die Erfindung betrifft neue m-Dihydroxybenzol-Derivate sowie diese Verbindungen enthaltende Mittel zum Färben von Keratinfasem.

Auf dem Gebiet der Färbung von Keratinfasem, insbesondere der Haarfärbung, haben Oxidationsfarbstoffe eine wesentliche Bedeutung erlangt. Die Färbung entsteht hierbei durch Reaktion bestimmter Entwicklersubstanzen mit bestimmten Kupplersubstanzen in Gegenwart eines geeigneten Oxidationsmittels. Als Entwicklersubstanzen werden hierbei insbesondere 2,5-Diaminotoluol, 2,5-Diaminophenylethylalkohol, p-Aminophenol, 1,4-Diaminobenzol und 4,5-Diamino-1-(2-hydroxyethyl)-pyrazol eingesetzt, während als Kupplersubstanzen beispielsweise Resorcin, 2-Methyl-resorcin, 1-Naphthol, 3-Aminophenol, m-Phenylendiamin, 2-Amino-4-(2'-hydroxyethyl)amino-anisol, 1,3-Diamino-4-(2'hydroxyethoxy)benzol und 2,4-Diamino-5-fluor-toluol zu nennen sind.

An Oxidationsfarbstoffe, die zur Färbung menschlicher Haare verwendet werden, werden neben der Färbung in der gewünschten Intensität zahlreiche zusätzliche Anforderungen gestellt. So müssen die Farbstoffe in toxikologischer und dermatologischer Hinsicht unbedenklich sein und die erzielten Haarfärbungen eine gute Lichtechtheit, Dauerwellechtheit, Säureechtheit und Reibeechtheit aufweisen. Auf jeden Fall aber müssen solche Färbungen ohne Einwirkung von Licht, Reibung und chemischen Mitteln über einen Zeitraum von mindestens 4 bis 6 Wochen stabil bleiben. Außerdem ist es erforderlich, dass durch Kombination geeigneter Entwicklersubstanzen und Kupplersubstanzen eine breite Palette verschiedener Farbnuancen erzeugt werden kann.

Es wurde bereits versucht, die Eigenschaften von m-Dihydroxybenzolen durch die Einführung von Substituenten zu verbessern. So sind zum Beispiel aus der EP-OS 0 904 774 und der WO-OS 99/15148 in 4-Stellung substituierte Resorcine bekannt. Weiterhin ist aus der DE-OS 30 016 905, DE-OS 32 33 541; US-PS 3 893 803 und der DE-OS 198 59 800 die Verwendung von substituierten m-Dihydroxybenzolen in Oxidationshaarfärbemitteln bekannt. Mit den derzeit bekannten Färbemitteln ist es jedoch nicht möglich, die an ein Färbemittel gestellten Anforderungen in jeder Hinsicht zu erfüllen. Es besteht daher weiterhin ein Bedürfnis nach neuen Kupplersubstanzen, welche die vorgenannten Anforderung in besonderem Masse erfüllen.

Es wurde nun gefunden, dass bestimmte 1,3-Dihydroxy-benzol-Derivate gemäß der allgemeinen Formel (I) die an Kupplersubstanzen gestellten Anforderungen in besonders hohem Masse erfüllen und mit den meisten bekannten Entwicklersubstanzen farbstarke Farbnuancen erhalten werden, welche außerordentlich farbintensiv, lichtecht und waschecht sind.

Gegenstand der vorliegende Erfindung sind daher neue 1,3-Dihydroxy-4-heteroaryl-benzol-Derivate der allgemeinen Formel (I) oder deren physiologisch verträglichen, wasserlöslichen Salze, 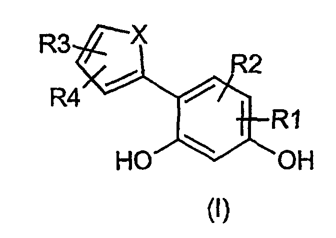 worin
**X** gleich Sauerstoff, Schwefel oder N-R5 ist;
**R1** und **R2** gleich oder verschieden sein können und unabhängig voneinander gleich Wasserstoff, einem Halogenatom (F, Cl, Br, J), einer Hydroxygruppe, einer C₁-C₄-Alkoxygruppe, einer C₁-C₄-Hydroxyalkoxygruppe, einer C₁-C₆-Alkylgruppe, einer C₁-C₄-Alkylthioethergruppe, einer Trifluormethangruppe, einer -Si(CH₃)₃-Gruppe, einer C₁-C₄-Hydroxyalkylgruppe oder einer C₂-C₄-Dihydroxyalkylgruppe ist;
**R3** und **R4** gleich oder verschieden sein können und unabhängig voneinander gleich unabhänging voneinander gleich Wasserstoff, einer Hydroxygruppe, einem Halogenatom, einer Cyanogruppe, einer C₁-C₄-Alkoxygruppe, einer C₁-C₆-Alkylgruppe, einer C₁-C₄-Alkylthioethergruppe, einer Mercaptogruppe, einer Nitrogruppe, einer Aminogruppe, einer C₁-C₄-Alkylaminogruppe, einer Di(C₁-C₄)alkylaminogruppe, einer -C(O)H-Gruppe, einer -C(O)CH₃-Gruppe, einer -C(O)CF₃-Gruppe, einer -Si(CH₃)₃-Gruppe, einer C₁-C₄-Hydroxyalkylgruppe, einer C₂-C₄-Dihydroxyalkylgruppe sind; und
**R5** gleich Wasserstoff, einer C₁-C₆-Alkylgruppe, einer C₂-C₄-Hydroxyalkylgruppe, einer Phenylgruppe oder einer Acetylgruppe ist.

Als Verbindungen der Formeln (I) können beispielweise genannt werden: 1,3-Dihydroxy-4-(3-chlor-thiophen-2-yl)-benzol, 1,3-Dihydroxy-4-(3-methylthiophen-2-yl)-benzol, 1,3-Dihydroxy-4-(3-nitro-thiophen-2-yl)-benzol, 1,3-Dihydroxy-4-(4-amino-thiophen-2-yl)-benzol, 1,3-Dihydroxy-4-(4-chlorthiophen-2-yl)-benzol, 1,3-Dihydroxy-4-(4-methyl-thiophen-2-yl)-benzol, 1,3-Dihydroxy-4-(4-nitro-thiophen-2-yl)-benzol, 1,3-Dihydroxy-4-(5-nitrothiophen-2-yl)-benzol,1,3-Dihydroxy-4-(5-amino-thiophen-2-yl)-benzol, 1,3-Dihydroxy-4-(5-chlor-thiophen-2-yl)-benzol, 1,3-Dihydroxy-4-(5-methylthiophen-2-yl)-benzol, 1,3-Dihydroxy-4-(5-nitro-thiophen-2-yl)-benzol, 1,3,5-Trihydroxy-4-(thiophen-2-yl)-benzol, 1,2,3-Trihydroxy-4-(thiophen-2-yl)-benzol, 1,3,6-Trihydroxy-4-(thiophen-2-yl)-benzol, 1,3-Dihydroxy-2-methyl-4-(thiophen-2-yl)-benzol, 1,3-Dihydroxy-5-methyl-4-(thiophen-2-yl)-benzol, 1,3-Dihydroxy-6-methyl-4-(thiophen-2-yl)-benzol, 1,3-Dihydroxy-2-chlor-4-(thiophen-2-yl)-benzol, 1,3-Dihydroxy-5-chlor-4-(thiophen-2-yl)-benzol, 1,3-Dihydroxy-6-chlor-4-(thiophen-2-yl)-benzol, 1,3-Dihydroxy-2-methoxy-4-(thiophen-2-yl)-benzol, 1,3-Dihydroxy-5-methoxy-4-(thiophen-2-yl)-benzol und 1,3-Dihydroxy-6-methoxy-4-(thiophen-2-yl)-benzol.

Bevorzugt sind Verbindungen der Formel (I), bei denen (i) eine oder mehrere der Restgruppen **R1, R2, R3** und **R4** gleich Wasserstoff sind und **X** gleich Schwefel oder Sauerstoff ist oder (ii) **R1** und **R2** gleichzeitig Wasserstoff bedeuten und **X** gleich Schwefel ist.

Besonders bevorzugt sind die folgenden Verbindungen der Formel (I) : 1,3-Dihydroxy-4-(thiophen-2-yl)-benzol, 1,3-Dihydroxy-4-(5-nitro-thiophen-2-yl)-benzol, 1,3-Dihydroxy-4-(3-methyl-thiophen-2-yl)-benzol und 1,3-Dihydroxy-4-(furan-2-yl)-benzol sowie deren physiologisch verträglichen Salze.

Die Herstellung der erfindungsgemäßen 1,3-Dihydroxy-benzol-Derivate der Formel (I) kann unter Verwendung von bekannten Syntheseverfahren erfolgen. Die Synthese der erfindungsgemäßen Verbindungen kann durch eine Palladium(0) katalysierte Kupplung eines substituierten Benzols der Formel (II) mit einer Heteroarylverbindung der Formel (III) und anschließende Abspaltung der Schutzgruppe erfolgen; 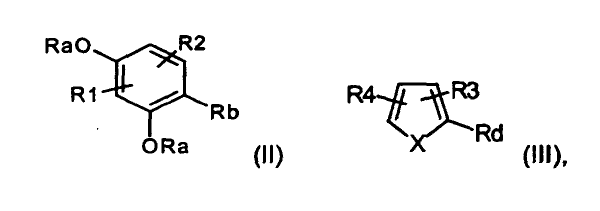 wobei
**Rb** die Bedeutung Halogen (F, Cl, Br, J) und **Rd** die Bedeutung B(OH)₂ hat, beziehungsweise **Rb** die Bedeutung B(OH)₂ und **Rd** die Bedeutung Halogen (F, Cl, Br, J) hat;
**Ra** für eine Schutzgruppe steht, wie sie zum Beispiel in dem Kapitel "Protective Groups" in Organic Synthesis, Kapitel 3, Wiley Interscience, 1991 beschrieben wird, und
**X, R1, R2, R3** und **R4** die in der Formel (I) genannte Bedeutung haben.

Die 1,3-Dihydroxybenzol-Derivate der Formel (I) sind gut in Wasser löslich und ermöglichen Färbungen mit ausgezeichneter Farbintensität und Farbechtheit, insbesondere was die Lichtechtheit, Waschechtheit und Reibeechtheit anbetrifft. Sie weisen weiterhin eine ausgezeichnete Lagerstabilität, insbesondere als Bestandteil der nachstehend beschriebenen Oxidationsfärbemitteln, auf.

Ein weiterer Gegenstand der vorliegenden Erfindung sind daher Mittel zum oxidativen Färben von Keratinfasern, wie zum Beispiel Haaren, Pelzen, Federn oder Wolle, insbesondere menschlichen Haaren, auf der Basis einer Entwicklersubstanz-Kupplersubstanz-Kombination, welche mindestens ein 1,3-Dihydroxybenzol-Derivat der Formel (I) enthalten.

Die Verbindungen der Formel (I) können sowohl als freie Phenole als auch in Form ihrer physiologisch verträglichen Salze mit anorganischen oder organischen Basen, wie zum Beispiel Natriumhydroxid, Kaliumhydroxid, Natriumcarbonat oder Tetrabutylammoniumhydroxid, eingesetzt werden.

Die 1,3-Dihydroxybenzol-Derivate der Formel (I) sind in dem erfindungsgemäßen Färbemittel in einer Gesamtmenge von etwa 0,005 bis 20 Gewichtsprozent enthalten, wobei eine Menge von etwa 0,01 bis 5 Gewichtsprozent und insbesondere 0,1 bis 2,5 Gewichtsprozent bevorzugt ist.

Als Entwicklersubstanzen kommen vorzugsweise 1,4-Diamino-benzol (p-Phenylendiamin), 1,4-Diamino-2-methyl-benzol (p-Toluylendiamin), 1,4-Diamino-2,6-dimethyl-benzol, 1,4-Diamino-3,5-diethyl-benzol, 1,4-Diamino-2,5-dimethyl-benzol, 1,4-Diamino-2,3-dimethyl-benzol, 2-Chlor-1,4-diaminobenzol, 1,4-Diamino-2-(thiophen-2-yl)benzol, 1,4-Diamino-2-(thiophen-3-yl)benzol, 1,4-Diamino-2-(pyridin-3-y1)benzol, 2,5-Diaminobiphenyl, 1,4-Diamino-2-methoxymethyl-benzol, 1,4-Diamino-2-aminomethyl-benzol, 1,4-Diamino-2-hydroxymethyl-benzol, 1,4-Diamino-2-(2-hydroxyethoxy)-benzol, 2-(2-(Acetylamino)ethoxy)-1,4-diamino-benzol, 4-Phenylamino-anilin, 4-Dimethylamino-anilin, 4-Diethylamino-anilin, 4-Dipropylamino-anilin, 4-[Ethyl(2-hydroxyethyl)-amino]-anilin, 4-[Di(2-hydroxyethyl)amino]-anilin, 4-[Di(2-hydroxyethyl)-amino]-2-methyl-anilin, 4-[(2-Methoxyethyl)amino]-anilin, 4-[(3-Hydroxypropyl)amino]-anilin, 4-[(2,3-Dihydroxypropyl)amino]-anilin, 1,4-Diamino-2-(1-hydroxyethyl)-benzol, 1,4-Diamino-2-(2-hydroxyethyl)-benzol, 1,4-Diamino-2-(1-methylethyl)-benzol, 1,3-Bis[(4-aminophenyl)(2-hydroxyethyl)amino]-2-propanol, 1,4-Bis[(4-aminophenyl)amino]-butan, 1,8-Bis(2,5-diaminophenoxy)-3,6-dioxaoctan, 4-Amino-phenol, 4-Amino-3-methyl-phenol, 4-Amino-3-(hydroxymethyl)-phenol, 4-Amino-3-fluorphenol, 4-Methylamino-phenol, 4-Amino-2-(aminomethyl)-phenol, 4-Amino-2-(hydroxymethyl)-phenol, 4-Amino-2-fluor-phenol, 4-Amino-2-[(2-hydroxyethyl)-amino]methyl-phenol, 4-Amino-2-methyl-phenol, 4-Amino-2-(methoxymethyl)-phenol, 4-Amino-2-(2-hydroxyethyl)-phenol, 5-Amino-salicylsäure, 2,5-Diamino-pyridin, 2,4,5,6-Tetraamino-pyrimidin, 2,5,6-Triamino-4-(1H)-pyrimidon, 4,5-Diamino-1-(2-hydroxyethyl)-1Hpyrazol, 4,5-Diamino-1-(1-methylethyl)-1H-pyrazol, 4,5-Diamino-1-[(4-methylphenyl)methyl]-1H-pyrazol, 1-[(4-Chlorphenyl)methyl]-4,5-diamino-1H-pyrazol, 4,5-Diamino-1-methyl-1H-pyrazol, 2-Amino-phenol, 2-Amino-6-methyl-phenol, 2-Amino-5-methyl-phenol und 1,2,4-Trihydroxy-benzol in Betracht.

Weiterhin kann das erfindungsgemäße Färbemittel zusätzlich zu den Verbindungen der Formel (I) noch weitere bekannte Kupplersubstanzen, beispielsweise N-(3-Dimethylamino-phenyl)-hamstoff, 2,6-Diamino-pyridin, 2-Amino-4-[(2-hydroxyethyl)amino]-anisol, 2,4-Diamino-1-fluor-5-methylbenzol, 2,4-Diamino-1-methoxy-5-methyl-benzol, 2,4-Diamino-1-ethoxy-5-methyl-benzol, 2,4-Diamino-1-(2-hydroxyethoxy)-5-methyl-benzol, 2,4-Di[(2-hydroxyethyl)amino]-1,5-dimethoxy-benzol, 2,3-Diamino-6-methoxypyridin, 3-Amino-6-methoxy-2-(methylamino)-pyridin, 2,6-Diamino-3,5-dimethoxy-pyridin, 3,5-Diamino-2,6-dimethoxy-pyridin, 1,3-Diamino-benzol, 2,4-Diamino-1-(2-hydroxyethoxy)-benzol, 1,3-Diamino-4-(2,3-dihydroxypropoxy)-benzol, 1,3-Diamino-4-(3-hydroxypropoxy)-benzol, 1,3-Diamino-4-(2-methoxyethoxy)-benzol, 2,4-Diamino-1,5-di(2-hydroxyethoxy)-benzol, 1-(2-Aminoethoxy)-2,4-diamino-benzol, 2-Amino-1-(2-hydroxyethoxy)-4-methylamino-benzol, 2,4-Diaminophenoxy-essigsäure, 3-[Di(2-hydroxyethyl)amino]-anilin, 4-Amino-2-di[(2-hydroxyethyl)amino]-1-ethoxy-benzol, 5-Methyl-2-(1-methylethyl)-phenol, 3-[(2-Hydroxyethyl)amino]-anilin, 3-[(2-Aminoethyl)amino]-anilin, 1,3-Di(2,4-diaminophenoxy)-propan, Di(2,4-diaminophenoxy)-methan, 1,3-Diamino-2,4-dimethoxy-benzol, 2,6-Bis(2-hydroxyethyl)amino-toluol, 4-Hydroxyindol, 3-Dimethylamino-phenol, 3-Diethylamino-phenol, 5-Amino-2-methyl-phenol, 5-Amino-4-fluor-2-methyl-phenol, 5-Amino-4-methoxy-2-methyl-phenol, 5-Amino-4-ethoxy-2-methyl-phenol, 3-Amino-2,4-dichlor-phenol, 5-Amino-2,4-dichlor-phenol, 3-Amino-2-methyl-phenol, 3-Amino-2-chlor-6-methyl-phenol, 3-Amino-phenol, 2-[(3-Hydroxyphenyl)-amino]-acetamid, 5-[(2-Hydroxyethyl)amino]-4-methoxy-2-methyl-phenol, 5-[(2-Hydroxyethyl)amino]-2-methyl-phenol, 3-[(2-Hydroxyethyl)amino]-phenol, 3-[(2-Methoxyethyl)amino]-phenol, 5-Amino-2-ethyl-phenol, 5-Amino-2-methoxy-phenol, 2-(4-Amino-2-hydroxyphenoxy)-ethanol, 5-[(3-Hydroxypropyl)amino]-2-methyl-phenol, 3-[(2,3-Dihydroxypropyl)-amino]-2-methyl-phenol, 3-[(2-Hydroxyethyl)amino]-2-methyl-phenol, 2-Amino-3-hydroxy-pyridin, 2,6-Dihydroxy-3,4-dimethylpyridin, 5-Amino-4-chlor-2-methyl-phenol, 1-Naphthol, 2-Methyl-1-naphthol, 1,5-Dihydroxy-naphthalin, 1,7-Dihydroxy-naphthalin, 2,3-Dihydroxy-naphthalin, 2,7-Dihydroxy-naphthalin, 2-Methyl-1-naphthol-acetat, 1,3-Dihydroxy-benzol, 1-Chlor-2,4-dihydroxy-benzol, 2-Chlor-1,3-dihydroxy-benzol, 1,2-Dichlor-3,5-dihydroxy-4-methyl-benzol, 1,5-Dichlor-2,4-dihydroxybenzol, 1,3-Dihydroxy-2-methyl-benzol, 3,4-Methylendioxy-phenol, 3,4-Methylendioxy-anilin, 5-[(2-Hydroxyethyl)amino]-1,3-benzodioxol, 6-Brom-1-hydroxy-3,4-methylendioxy-benzol, 3,4-Diamino-benzoesäure, 3,4-Dihydro-6-hydroxy-1,4(2H)-benzoxazin, 6-Amino-3,4-dihydro-1,4(2H)-benzoxazin, 3-Methyl-1-phenyl-5-pyrazolon, 5,6-Dihydroxy-indol, 5,6-Dihydroxy-indolin, 5-Hydroxy-indol, 6-Hydroxy-indol, 7-Hydroxy-indol und 2,3-Indolindion, enthalten.

Die Kupplersubstanzen und Entwicklersubstanzen können in dem erfindungsgemäßen Färbemittel jeweils einzeln oder im Gemisch miteinander enthalten sein, wobei die Gesamtmenge an Kupplersubstanzen und Entwicklersubstanzen in dem erfindungsgemäßen Färbemittel (bezogen auf die Gesamtmenge des Färbemittels) jeweils etwa 0,005 bis 20 Gewichtsprozent, vorzugsweise etwa 0,01 bis 5 Gewichtsprozent und insbesondere 0,1 bis 2,5 Gewichtsprozent, beträgt.

Die Gesamtmenge der in dem hier beschriebenen Färbemittel enthaltenen Entwicklersubstanz-Kupplersubstanz-Kombination beträgt vorzugsweise etwa 0,01 bis 20 Gewichtsprozent, wobei eine Menge von etwa 0,02 bis 10 Gewichtsprozent und insbesondere 0,2 bis 6 Gewichtsprozent besonders bevorzugt ist. Die Entwicklersubstanzen und Kupplersubstanzen werden im allgemeinen in etwa äquimolaren Mengen eingesetzt; es ist jedoch nicht nachteilig, wenn die Entwicklersubstanzen diesbezüglich in einem gewissen Überschuß oder Unterschuß vorhanden sind.

Weiterhin kann das erfindungsgemäße Färbemittel zusätzlich andere Farbkomponenten, beispielsweise 6-Amino-2-methylphenol und 2-Amino-5-methylphenol, sowie ferner übliche direktziehende Farbstoffe aus der Gruppe der sauren oder basischen Farbstoffe, der Triphenylmethanfarbstoffe, der aromatischen Nitrofarbstoffe, der Azofarbstoffe und der Dispersionsfarbstoffe, enthalten. Die erfindungsgemäßem Färbemittel können diese Farbkomponenten in einer Menge von etwa 0,1 bis 4 Gewichtsprozent enthalten.

Selbstverständlich können die zusätzlichen Kupplersubstanzen sowie die Entwicklersubstanzen und die anderen Farbkomponenten, sofern es Basen sind, auch in Form der physiologisch verträglichen Salze mit organischen oder anorganischen Säuren, wie beispielsweise Salzsäure oder Schwefel-säure, beziehungsweise - sofern sie aromatische OH-Gruppen besitzen - in Form der Salze mit Basen, zum Beispiel als Alkaliphenolate, eingesetzt werden.

Darüber hinaus können in den Färbemitteln, falls diese zur Färbung von Haaren verwendet werden sollen, noch weitere übliche kosmetische Zusätze, beispielsweise Antioxidantien wie Ascorbinsäure, Thioglykolsäure oder Natriumsulfit, sowie Parfümöle, Komplexbildner, Netzmittel, Emulgatoren, Verdicker und Pflegestoffe enthalten sein.

Die Zubereitungsform des erfindungsgemäßen Färbemittels kann beispielsweise eine Lösung, insbesondere eine wässrige oder wässrigalkoholische Lösung sein. Die besonders bevorzugten Zubereitungsformen sind jedoch eine Creme, ein Gel oder eine Emulsion. Ihre Zusammensetzung stellt eine Mischung der Farbstoffkomponenten mit den für solche Zubereitungen üblichen Zusätzen dar.

Ubliche Zusätze in Lösungen, Cremes, Emulsionen oder Gelen sind zum Beispiel Lösungsmittel wie Wasser, niedere aliphatische Alkohole, beispielsweise Ethanol, Propanol oder Isopropanol, Glycerin oder Glykole wie 1,2-Propylenglykol, weiterhin Netzmittel oder Emulgatoren aus den Klassen der anionischen, kationischen, amphoteren oder nichtionogenen oberflächenaktiven Substanzen wie zum Beispiel Fettalkoholsulfate, oxethylierte Fettalkoholsulfate, Alkylsulfonate, Alkylbenzolsulfonate, Alkyltrimethylammoniumsalze, Alkylbetaine, oxethylierte Fettalkohole, oxethylierte Nonylphenole, Fettsäurealkanolamide und oxethylierte Fettsäureester ferner Verdicker wie höhere Fettalkohole, Stärke, Cellulosederivate, Petrolatum, Paraffinöl und Fettsäuren, sowie außerdem Pflegestoffe wie kationische Harze, Lanolinderivate, Cholesterin, Pantothensäure und Betain. Die erwähnten Bestandteile werden in den für solche Zwecke üblichen Mengen verwendet, zum Beispiel die Netzmittel und Emulgatoren in Konzentrationen von etwa 0,5 bis 30 Gewichtsprozent, die Verdicker in einer Menge von etwa 0,1 bis 30 Gewichtsprozent und die Pflegestoffe in einer Konzentration von etwa 0,1 bis 5 Gewichtsprozent.

Je nach Zusammensetzung kann das erfindungsgemäße Färbemittel schwach sauer, neutral oder alkalisch reagieren. Insbesondere weist es einen pH-Wert von 6,5 bis 11,5 auf, wobei die basische Einstellung vorzugsweise mit Ammoniak erfolgt. Es können aber auch organische Amine, zum Beispiel Monoethanolamin und Triethanolamin, oder auch anorganische Basen wie Natriumhydroxid und Kaliumhydroxid Verwendung finden. Für eine pH-Einstellung im sauren Bereich kommen anorganische oder organische Säuren, zum Beispiel Phosphorsäure, Essigsäure Zitronensäure oder Weinsäure, in Betracht.

Für die Anwendung zur oxidativen Färbung von Haaren vermischt man das vorstehend beschriebene Färbemittel unmittelbar vor dem Gebrauch mit einem Oxidationsmittel und trägt eine für die Haarfärbebehandlung ausreichende Menge, je nach Haarfülle, im allgemeinen etwa 60 bis 200 Gramm, dieses Gemisches auf das Haar auf.

Als Oxidationsmittel zur Entwicklung der Haarfärbung kommen hauptsächlich Wasserstoffperoxid oder dessen Additionsverbindungen an Harnstoff, Melamin, Natriumborat oder Natriumcarbonat in Form einer 3-bis 12prozentigen, vorzugsweise 6prozentigen, wässrigen Lösung, aber auch Luftsauerstoff in Betracht. Wird eine 6prozentige Wasserstoffperoxid-Lösung als Oxidationsmittel verwendet, so beträgt das Gewichtsverhältnis zwischen Haarfärbemittel und Oxidationsmittel 5:1 bis 1:2, vorzugeweise jedoch 1:1. Größere Mengen an Oxidationsmittel werden vor allem bei höheren Farbstoffkonzentrationen im Haarfärbemittel, oder wenn gleichzeitig eine stärkere Bleichung des Haares beabsichtigt ist, verwendet. Man läßt das Gemisch bei 15 bis 50 Grad Celsius etwa 10 bis 45 Minuten lang, vorzugsweise 30 Minuten lang, auf das Haar einwirken, spült sodann das Haar mit Wasser aus und trocknet es. Gegebenenfalls wird im Anschluß an diese Spülung mit einem Shampoo gewaschen und eventuell mit einer schwachen organischen Säure, wie zum Beispiel Zitronensäure oder Weinsäure, nachgespült. Anschließend wird das Haar getrocknet.

Das erfindungsgemäße Färbemittel mit einem Gehalt an 1,3-Dihydroxybenzol-Derivaten der Formel (I) als Kupplersubstanz ermöglicht Färbungen mit ausgezeichneter Farbechtheit, insbesondere was die Lichtechtheit, Waschechtheit und Reibeechtheit anbetrifft. Hinsichtlich der färberischen Eigenschaften bietet das erfindungsgemäße Färbemittel je nach Art und Zusammensetzung der Farbkomponenten eine breite Palette verschiedener Farbnuancen, welche sich von blonden über braune, purpurne, violette bis hin zu blauen und schwarzen Farbtönen erstreckt. Hierbei zeichnen sich die Farbtöne durch ihre besondere Farbintensität aus. Die sehr guten färberischen Eigenschaften des Färbemittels gemäß der vorliegenden Anmeldung zeigen sich weiterhin darin, daß dieses Mittel insbesondere auch eine Anfärbung von ergrauten, chemisch nicht vorgeschädigten Haaren problemlos und mit guter Deckkraft ermöglicht.

Die nachfolgenden Beispiele sollen den Gegenstand der Erfindung näher erläutern, ohne ihn darauf zu beschränken.

### Beispiele

### Beispiel 1: Synthese von 1,3-Dihydroxybenzolen (Alfgemeine Synthesevorschrift)

### A. Synthese von 2-[2-Brom-5-(tetrahydro-2H-pyran-2-yloxy)phenoxy]-tetrahydro-2H-pyran

Zu einer Lösung von 9,5 g (40 mmol) 4-Brom-1,3-dihydroxybenzol und 5 g (20 mmol) Toluol-4-sulfonsäure-Pyridinsalz in 50 ml Dichlormethan werden unter Argon 9 g (105 mmol) 3,4-Dihydro-2-H-pyran getropft. Das Reaktionsgemisch wird 18 Stunden lang gerührt. Anschließend wird die Reaktionsmischung in 100 ml Essigsäureethylester gegossen, die organische Phase mit verdünnter Natronlauge extrahiert und sodann mit Magnesiumsulfat getrocknet. Das Lösungsmittel wird am Rotationsverdampfer abdestilliert und der Rückstand an Kieselgel mit Petrolether/Essigsäureethylester (7:1) gereinigt.
Es werden 9,5 g (54% der Theorie) 2-[2-Brom-5-(tetrahydro-2H-pyran-2-yloxy)phenoxy]tetrahydro-2H-pyran erhalten.

### B. Synthese von 2-[2-(4,4,5,5-tetramethyl-[1,3,2]dioxaborolan-2-yl)-5-(tetrahydro-2H-pyran-2-yl-oxy)phenoxy]-tetrahydro-2H-pyran

Eine Mischung von 5,5 g (154 mmol) 2-[2-Brom-5-(tetrahydro-2H-pyran-2-yloxy)phenoxy]tetrahydro-2H-pyran aus Stufe **A**, 9 g (355 mmol) 4,4,5,5,4',4',5',5'-Octamethyl-[2,2']bi[[1,3,2]dioxaborinanyl], 1,5 g (2,2 mmol) Dichloro(1,1'-bis(diphenyl-phosphino)ferrocene)palladium (PdCl₂(dppf)) und 6,2 g (63,2 mmol) Kaliumacetat wird unter Argon mit 210 ml entgastem Dioxan versetzt. Das Gemisch wird 24 Stunden lang bei 80 °C gerührt, sodann auf Wasser gegossen und mit Essigsäureethylester extrahiert. Die organische Phase wird anschließend mit einer gesättigten wässerigen Kochsalz-Lösung gewaschen, über Natriumsulfat getrocknet und nach Filtration eingeengt. Das erhaltene Rohprodukt wird an desaktiviertem Kieselgel mit einem Gradienten von Hexan (100%) über Hexan/Essigsäureethylester (9:1 bis 3:2) gereinigt.
Es werden 4,70 g (77% der Theorie) 2-[2-(4,4,5,5-tetramethyl-[1,3,2]dioxaborolan-2-yl)-5-(tetrahydro-2H-pyran-2-yl-oxy)phenoxy]-tetrahydro-2H-pyran erhalten.

### C. Synthese der 1,3-Dihydroxy-benzole der Formel (I)

0,09 g (0,2 mmol) 2- [2-(4,4,5,5-tetramethyl-[1,3,2]dioxaborolan-2-yl)-5-(tetrahydro-2H-pyran-2-yl-oxy)phenoxy]- tetrahydro-2H-pyran aus Stufe B und 0,4 mmol des entsprechenden Bromderivates werden unter Argon in 10 ml 1,2-Dimethoxyethan gelöst. Anschließend werden 0,01 g (0,005 mmol) Tetrakis-(triphenylphosphin)-palladium und 0,26 ml 2N Kaliumcarbonat-Lösung zugegeben und die Reaktionsmischung auf 80 °C erwärmt. Nach Beendigung der Reaktion wird die Reaktionsmischung in 10 ml Essigsäureethylester gegossen, die organische Phase mit verdünnter Natronlauge extrahiert und sodann mit Magnesiumsulfat getrocknet. Das Lösungsmittel wird am Rotationsverdampfer abdestilliert und der Rückstand an Kieselgel mit Petrolether/Essigsäureethylester (9:1) gereinigt.

### a. 1,3-Dihydroxy-4-(thiophenyl-2-yl)-benzol

Verwendetes Bromderivat: 2-Brom-thiophen
Massenspektrum: MH+ 192 (100)

### b. 1,3-Dihydroxy-4-(5-nitro-thiophen-2-yl)-benzol

Verwendetes Bromderivat: 2-Brom-5-nitro-thiophen
Massenspektrum: MH+ 237 (100)

### c. 1,3-Dihydroxy-4-(3-methyl-thiophen-2-yl)-benzol

Verwendetes Bromderivat: 2-Brom-3-methyl-thiophen
   Massenspektrum: MH+ 206 (100)

### Beispiel 2 bis 4: Haarfärbemittel

Es werden Haarfärbelösungen der folgenden Zusammensetzung hergestellt:

| | |
|---|---|
| 1,25 mmol | Kupplersubstanz der Formel (I) gemäß Tabelle 1 |
| 1,25 mmol | Entwicklersubstanz gemäß Tabelle 1 |
| 1,0 g | Kaliumoleat (8prozentige wässrige Lösung) |
| 1,0 g | Ammoniak (22prozentige wässrige Lösung) |
| 1,0 g | Ethanol |
| 0,3 g | Ascorbinsäure |
| ad 100,0 g | Wasser |

50 g der vorstehenden Färbelösung werden unmittelbar vor der Anwendung mit 50 g einer 6prozentigen wässrigen Wasserstoffperoxidlösung vermischt. Anschließend wird das Gemisch auf gebleichte Haare aufgetragen. Nach einer Einwirkungszeit von 30 Minuten bei 40 °C wird das Haar mit Wasser gespült, mit einem handelsüblichen Shampoo . gewaschen und getrocknet. Die resultierenden Färbungen sind in Tabelle 1 zusammengefaßt.

**Tabelle 1:**

| **Beispiel Nr.** | **Kupplersubstanz der Formel (I)/(Ia)** | **Kupplersubstanz** | | | |
|---|---|---|---|---|---|
| | | **I. 1,4-Di- amino- benzol** | **II. 2,5- Diamino- toluol-sulfat** | **III. 2,5-Diamino- phenyl- ethanol- sulfat** | **IV. 4,5-Diamino-1- (2'-hydroxy- ethyl)-pyrazol- sulfat** |
| **2** | gemäß Beispiel **1a** | dunkelbraun | dunkelbraun | dunkelbraun | purpur |
| **3** | gemäß Beispiel **1b** | braun | mittelblond | mittelblond | purpur |
| **4** | gemäß Beispiel **1c** | braun | mittelblond | mittelblond | purpur |

### Beispiel 5 bis 14: Haarfärbemittel

Es werden Haarfärbelösungen der folgenden Zusammensetzung hergestellt:

| | |
|---|---|
| X g | 1,3-Dihydroxy-4-(thiophen-2-yl)-benzol (Kupplersubstanz **K2**) |
| U g | Entwicklersubstanz **E8** bis **E15** gemäß Tabelle 2 |
| Y g | Kupplersubstanz **K11** bis **K33** gemäß Tabelle 3 |
| 10,0 g | Kaliumoleat (8prozentige wässrige Lösung) |
| 10,0 g | Ammoniak (22prozentige wässrige Lösung) |
| 10,0 g | Ethanol |
| 0,3 g | Ascorbinsäure |
| ad 100,0 g | Wasser |

30 g der vorstehenden Färbelösung werden unmittelbar vor der Anwendung mit 30 g einer 6prozentigen wässrigen Wasserstoffperoxidlösung vermischt. Anschließend wird das Gemisch auf gebleichte Haare aufgetragen. Nach einer Einwirkungszeit von 30 Minuten bei 40 °C wird das Haar mit Wasser gespült, mit einem handelsüblichen Shampoo gewaschen und getrocknet. Die Färbeergebnisse sind in Tabelle 5 zusammengefasst.

**Tabelle 2:**

| **Entwicklersubstanzen** | |
|---|---|
| **E8** | 1,4-Diaminobenzol |
| **E9** | 2,5-Diamino-phenylethanol-sulfat |
| **E10** | 3-Methyl-4-amino-phenol |
| **E11** | 4-Amino-2-aminomethyl-phenol-dihydrochlorid |
| **E12** | 4-Amino-phenol |
| **E13** | N,N-Bis(2'-hydroxyethyl)-p-phenylendiamin-sulfat |
| **E14** | 4,5-Diamino-1-(2'-hydroxyethyl)-pyrazol-sulfat |
| **E15** | 2,5-Diaminotoluol-sulfat |

**Tabelle 3:**

| **Kupplersubstanzen** | |
|---|---|
| **K2** | 1,3-Dihydroxy-4-(thiophenyl-2-yl)-benzol |
| | |
| **K12** | 2-Amino-4-(2'-hydroxyethyl)amino-anisol-sulfat |
| **K13** | 1,3-Diamino-4-(2'-hydroxyethoxy)benzol-sulfat |
| **K18** | N-(3-Dimethylamino)phenylhamstoff |
| **K19** | 1,3-Bis(2,4-Diaminophenoxy)propan-tetrahydrochlorid |
| **K21** | 3-Amino-phenol |
| **K22** | 5-Amino-2-methyl-phenol |
| **K23** | 3-Amino-2-chlor-6-methyl-phenol |
| **K24** | 5-Amino-4-fluor-2-methyl-phenol-sulfat |
| **K25** | 1-Naphthol |
| **K26** | 1-Acetoxy-2-methyl-naphthalin |
| **K31** | 1,3-Dihydroxy-benzol |
| **K32** | 2-Methyl-1,3-dihydroxy-benzol |
| **K33** | 1-Chlor-2,4-dihydroxy-benzol |

**Tabelle 4:**

| **Direktziehende Farbstoffe** | |
|---|---|
| **D2** | 6-Chlor-2-ethylamino-4-nitro-phenol |
| **D3** | 2-Amino-6-chlor-4-nitro-phenol |

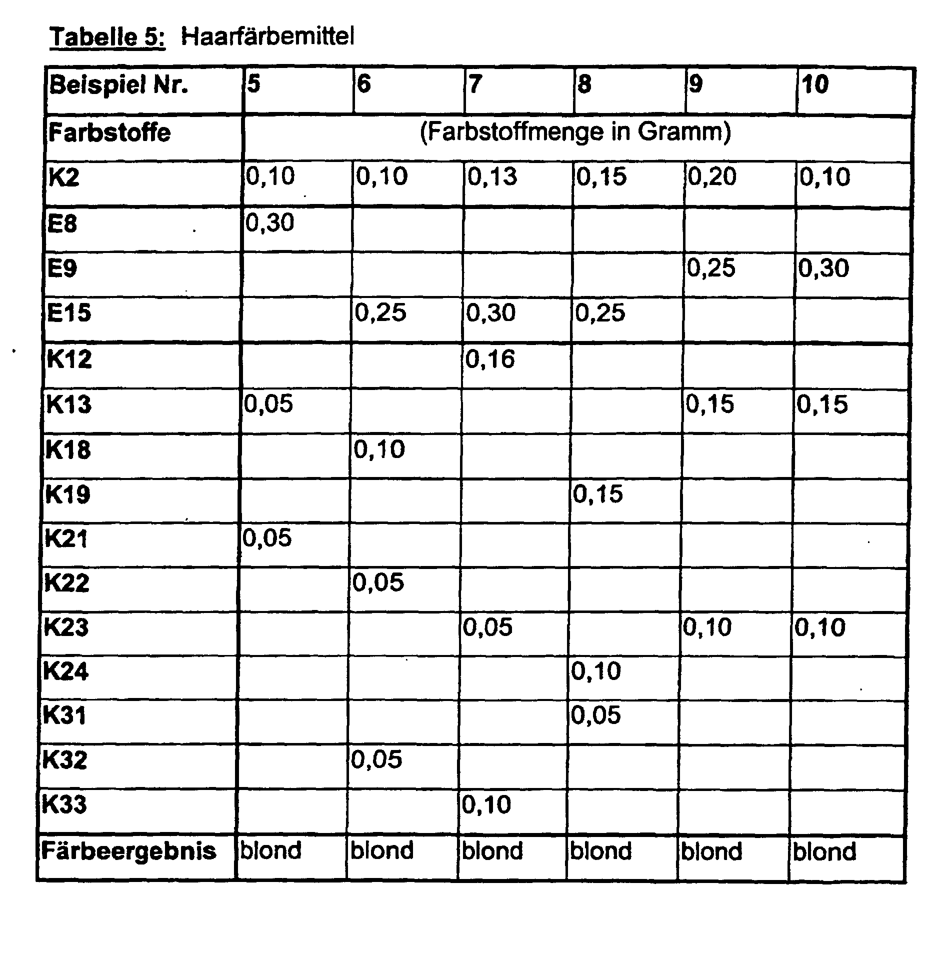

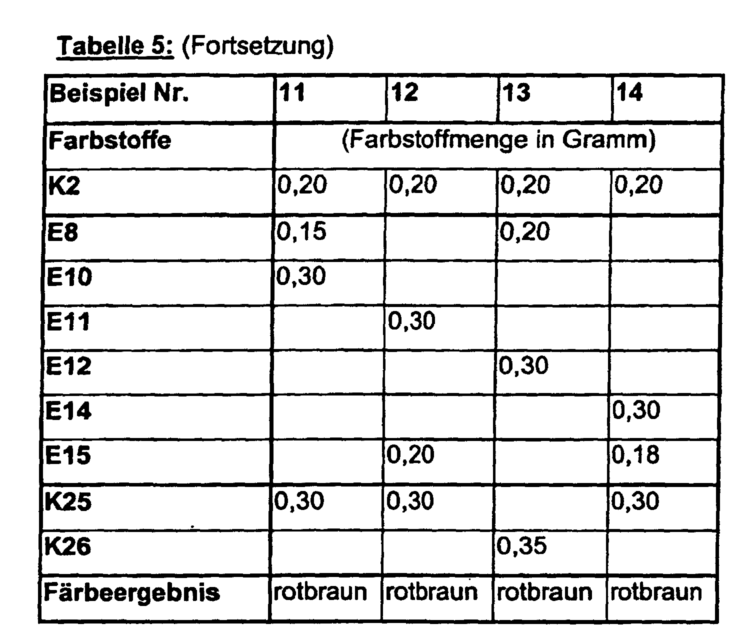

### Beispiel 15 bis 20: Haarfärbemittel

Es werden cremeförmige Farbträgermassen der folgenden Zusammensetzung hergestellt:

| | |
|---|---|
| X g | 1,3-Dihydroxy-4-(thiophen-2-yl)-benzol (Kupplersubstanz **K2**) |
| U g | Entwicklersubstanz **E8** bis **E15** gemäß Tabelle 2 |
| Y g | Kupplersubstanz **K11** bis **K33** gemäss Tabelle 3 |
| Z g | direktziehender Farbstoff **D2** bis **D3** gemäss Tabelle 4 |
| 15,0 g | Cetylalkohol |
| 0,3 g | Ascorbinsäure |
| 3,5 g | Natriumlaurylalkoholdiglycolethersulfat, 28prozentige wässrige Lösung |
| 3,0 g | Ammoniak, 22prozentige wässrige Lösung |
| 0,3 g | Natriumsulfit, wasserfrei |
| ad 100 g | Wasser |

30 g der vorstehenden Färbecreme werden unmittelbar vor der Anwendung mit 30 g einer 6prozentigen Wasserstoffperoxidlösung vermischt. Anschliessend wird das Gemisch auf das Haar aufgetragen. Nach einer Einwirkzeit von 30 Minuten bei 40 °C wird das Haar mit Wasser gespült, mit einem handelsüblichen Shampoo gewaschen und getrocknet. Die Färbeergebnisse sind in Tabelle 6 zusammengefasst.

**Tabelle 6:**

| Haarfärbemittel | | | | | | |
|---|---|---|---|---|---|---|
| **Beispiel Nr.** | **11** | **12** | **13** | **14** | **15** | **16** |
| **Farbstoffe** | (Farbstoffmenge in Gramm) | | | | | |
| **K2** | 1,10 | 1,10 | 0,6 | 0,20 | 0,40 | 0,40 |
| **E8** | 1,50 | | | | | |
| **E13** | | 1,60 | | | | 0,70 |
| **E15** | | | 1,80 | 0,70 | 0,70 | |
| **K12** | 0,60 | | | | | |
| **K13** | 0,70 | 1,50 | 1,25 | 0,18 | 0,18 | 0,18 |
| **K23** | | | 0,05 | 0,10 | 0,10 | 0,10 |
| **K31** | | | 0,60 | 0,20 | | |
| **D3** | | | | 0,10 | | |
| **D2** | | | | | 0,10 | 0,10 |
| **Färbeergebnis** | schwarz | schwarz | schwarz | braun | braun | braun |

Alle in der vorliegenden Anmeldung enthaltenen Prozentangaben stellen soweit nicht anders angegeben Gewichtsprozente dar.

## Patentansprüche

1. 1,3-Dihydroxy-4-heteroaryl-benzol-Derivat der allgemeinen Formel (I) oder dessen physiologisch verträglichen, wasserlöslichen Salze, 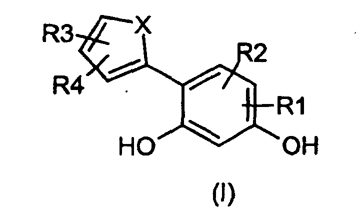 worin
**X** gleich Sauerstoff, Schwefel oder N-R5 ist;
**R1** und **R2** gleich oder verschieden sein können und unabhängig voneinander gleich Wasserstoff, einem Halogenatom, einer Hydroxygruppe, einer C₁-C₄-Alkoxygruppe, einer C₁-C₄-Hydroxyalkoxygruppe, einer C₁-C₆-Alkylgruppe, einer C₁-C₄-Alkylthioethergruppe, einer Trifluormethangruppe, einer -Si(CH₃)₃-Gruppe, einer C₁-C₄-Hydroxyalkylgruppe oder einer C₂-C₄-Dihydroxyalkylgruppe ist;
**R3** und **R4** gleich oder verschieden sein können und unabhängig voneinander gleich unabhänging voneinander gleich Wasserstoff, einer Hydroxygruppe, einem Halogenatom, einer Cyanogruppe, einer C₁-C₄-Alkoxygruppe, einer C₁-C₆-Alkylgruppe, einer C₁-C₄-Alkylthioethergruppe, einer Mercaptogruppe, einer Nitrogruppe, einer Aminogruppe, einer C₁-C₄-Alkylaminogruppe, einer Di(C₁-C₄)alkylaminogruppe, einer -C(O)H-Gruppe, einer -C(O)CH₃-Gruppe, einer-C(O)CF₃-Gruppe, einer -Si(CH₃)₃-Gruppe, einer C₁-C₄-Hydroxyalkylgruppe, einer C₂-C₄-Dihydroxyalkylgruppe sind; und
**R5** gleich Wasserstoff, einer C₁-C₆-Alkylgruppe, einer C₂-C₄-Hydroxyalkylgruppe, einer Phenylgruppe oder einer Acetylgruppe ist.

2. 1,3-Dihydroxy-4-heteroaryl-benzol-Derivat nach Anspruch 1, **dadurch gekennzeichnet, dass** es ausgewählt ist aus Verbindungen der Formel (I) bei denen (i) eine oder mehrere der Restgruppen **R1, R2, R3** und **R4** gleich Wasserstoff sind und **X** gleich Schwefel oder Sauerstoff ist oder (ii) **R1** und **R2** gleichzeitig Wasserstoff bedeuten und **X** gleich Schwefel ist.

3. 1,3-Dihydroxy-4-heteroaryl-benzol-Derivat der Formel (I) nach Anspruch 1 oder 2, **dadurch gekennzeichnet, dass** es ausgewählt ist aus der Gruppe bestehend aus 1,3-Dihydroxy-4-(thiophen-2-yl)-benzol, 1,3-Dihydroxy-4-(5-nitro-thiophen-2-yl)-benzol, 1,3-Dihydroxy-4-(3-methylthiophen-2-yl)-benzol und 1,3-Dihydroxy-4-(furan-2-yl)-benzol sowie deren physiologisch verträglichen Salze.

4. Mittel zum oxidativen Färben von Keratinfasem auf der Basis einer Entwicklersubstanz-Kupplersubstanz-Kombination, **dadurch gekennzeichnet, dass** es mindestens ein 1,3-Dihydroxy-4-heteroaryl-benzol-Derivat der Formel (I) nach einem der Ansprüche 1 bis 3 enthält

5. Mittel nach Anspruch 4, **dadurch gekennzeichnet, dass** die Verbindung der Formel (I) ausgewählt ist aus der Gruppe bestehend aus 1,3-Dihydroxy-4-(thiophen-2-yl)-benzol, 1,3-Dihydroxy-4-(5-nitro-thiophen-2-yl)-benzol, 1,3-Dihydroxy-4-(3-methyl-thiophen-2-yl)-benzol und 1,3-Dihydroxy-4-(furan-2-yl)-benzol sowie deren physiologisch verträglichen Salze.

6. Mittel nach Anspruch 4 oder 5, **dadurch gekennzeichnet, dass** das 1,3-Dihydroxy-4-heteroaryl-benzol der Formel (I) in einer Menge von 0,005 bis 20 Gewichtsprozent enthalten ist.

7. Mittel nach einem der Ansprüche 4 bis 6, **dadurch gekennzeichnet, dass** die Entwicklersubstanz ausgewählt ist aus der Gruppe bestehend aus 1,4-Diamino-benzol, 1,4-Diamino-2-methyl-benzol, 1,4-Diamino-2,6-dimethyl-benzol, 1,4-Diamino-3,5-diethyl-benzol, 1,4-Diamino-2,5-dimethyl-benzol, 1,4-Diamino-2,3-dimethyl-benzol, 2-Chlor-1,4-diaminobenzol, 1,4-Diamino-2-(thiophen-2-yl)benzol, 1,4-Diamino-2-(thiophen-3-yl)benzol, 1,4-Diamino-2-(pyridin-3-yl)benzol, 2,5-Diaminobiphenyl, 1,4-Diamino-2-methoxymethyl-benzol, 1,4-Diamino-2-aminomethyl-benzol, 1,4-Diamino-2-hydroxymethyl-benzol, 1,4-Diamino-2-(2-hydroxyethoxy)-benzol, 2-(2-(Acetylamino)ethoxy)-1,4-diamino-benzol, 4-Phenylamino-anilin, 4-Dimethylamino-anilin, 4-Diethylamino-anilin, 4-Dipropylamino-anilin, 4-[Ethyl(2-hydroxyethyl)amino]-anilin, 4-[Di(2-hydroxyethyl)amino]-anilin, 4-[Di(2-hydroxyethyl)amino]-2-methyl-anilin, 4-[(2-Methoxyethyl)amino]-anilin, 4-[(3-Hydroxypropyl)amino]-anilin, 4-[(2,3-Dihydroxypropyl)amino]-anilin, 1,4-Diamino-2-(1-hydroxyethyl)-benzol, 1,4-Diamino-2-(2-hydroxyethyl)-benzol, 1,4-Diamino-2-(1-methylethyl)-benzol, 1,3-Bis[(4-aminophenyl)(2-hydroxyethyl)amino]-2-propanol, 1,4-Bis[(4-aminophenyl)amino]-butan, 1,8-Bis(2,5-diaminophenoxy)-3,6-dioxaoctan, 4-Amino-phenol, 4-Amino-3-methyl-phenol, 4-Amino-3-(hydroxymethyl)-phenol, 4-Amino-3-fluor-phenol, 4-Methylamino-phenol, 4-Amino-2-(aminomethyl)-phenol, 4-Amino-2-(hydroxymethyl)-phenol, 4-Amino-2-fluor-phenol, 4-Amino-2-[(2-hydroxyethyl)-amino]methyl-phenol, 4-Amino-2-methyl-phenol, 4-Amino-2-(methoxymethyl)-phenol, 4-Amino-2-(2-hydroxyethyl)-phenol, 5-Amino-salicylsäure, 2,5-Diamino-pyridin, 2,4,5,6-Tetraamino-pyrimidin, 2,5,6-Triamino-4-(1H)-pyrimidon, 4,5-Diamino-1-(2-hydroxyethyl)-1Hpyrazol, 4,5-Diamino-1-(1-methylethyl)-1H-pyrazol, 4,5-Diamino-1-[(4-methylphenyl)methyl]-1H-pyrazol, 1-[(4-Chlorphenyl)methyl]-4,5-diamino-1 H-pyrazol, 4,5-Diamino-1-methyl-1H-pyrazol, 2-Amino-phenol, 2-Amino-6-methyl-phenol, 2-Amino-5-methyl-phenol und 1,2,4-Trihydroxy-benzol.

8. Mittel nach einem der Ansprüche 4 bis 7, **dadurch gekennzeichnet, dass** es zusätzlich zu den Verbindungen der Formel (I) noch weitere bekannte Kupplersubstanzen aus der Gruppe bestehend aus N-(3-Dimethylamino-phenyl)-hamstoff, 2,6-Diamino-pyridin, 2-Amino-4-[(2-hydroxyethyl)amino]-anisol, 2,4-Diamino-1-fluor-5-methyl-benzol, 2,4-Diamino-1-methoxy-5-methyl-benzol, 2,4-Diamino-1-ethoxy-5-methylbenzol, 2,4-Diamino-1-(2-hydroxyethoxy)-5-methyl-benzol, 2,4-Di[(2-hydroxyethyl)amino]-1,5-dimethoxy-benzol, 2,3-Diamino-6-methoxypyridin, 3-Amino-6-methoxy-2-(methylamino)-pyridin, 2,6-Diamino-3,5-dimethoxy-pyridin, 3,5-Diamino-2,6-dimethoxy-pyridin, 1,3-Diamino-benzol, 2,4-Diamino-1-(2-hydroxyethoxy)-benzol, 1,3-Diamino-4-(2,3-dihydroxypropoxy)-benzol, 1,3-Diamino-4-(3-hydroxypropoxy)-benzol, 1,3-Diamino-4-(2-methoxyethoxy)-benzol, 2,4-Diamino-1,5-di(2-hydroxyethoxy)-benzol, 1-(2-Aminoethoxy)-2,4-diamino-benzol, 2-Amino-1-(2-hydroxyethoxy)-4-methylamino-benzol, 2,4-Diaminophenoxyessigsäure, 3-[Di(2-hydroxyethyl)amino]-anilin, 4-Amino-2-di[(2-hydroxyethyl)amino]-1-ethoxy-benzol, 5-Methyl-2-(1-methylethyl)-phenol, 3-[(2-Hydroxyethyl)amino]-anilin, 3-[(2-Amino-ethyl)amino]-anilin, 1,3-Di(2,4-diaminophenoxy)-propan, Di(2,4-diamino-phenoxy)-methan, 1,3-Diamino-2,4-dimethoxy-benzol, 2,6-Bis(2-hydroxy-ethyl)amino-toluol, 4-Hydroxyindol, 3-Dimethylamino-phenol, 3-Diethyl-amino-phenol, 5-Amino-2-methyl-phenol, 5-Amino-4-fluor-2-methyl-phenol, 5-Amino-4-methoxy-2-methyl-phenol, 5-Amino-4-ethoxy-2-methyt-phenol, 3-Amino-2,4-dichlor-phenol, 5-Amino-2,4-dichlor-phenol, 3-Amino-2-methyl-phenol, 3-Amino-2-chlor-6-methyl-phenol, 3-Amino-phenol, 2-[(3-Hydroxyphenyl)-amino]-acetamid, 5-[(2-Hydroxyethyl)amino]-4-methoxy-2-methyl-phenol, 5-[(2-Hydroxyethyl)amino]-2-methyl-phenol, 3-[(2-Hydroxyethyl)amino]-phenol, 3-[(2-Methoxyethyl)amino]-phenol, 5-Amino-2-ethyl-phenol, 5-Amino-2-methoxy-phenol, 2-(4-Amino-2-hydroxyphenoxy)-ethanol, 5-[(3-Hydroxypropyl)amino]-2-methyl-phenol, 3-[(2,3-Dihydroxypropyl)-amino]-2-methyl-phenol, 3-[(2-Hydroxyethyl)-amino]-2-methyl-phenol, 2-Amino-3-hydroxy-pyridin, 2,6-Dihydroxy-3,4-dimethylpyridin, 5-Amino-4-chlor-2-methyl-phenol, 1-Naphthol, 2-Methyl-1-naphthol, 1,5-Dihydroxy-naphthalin, 1,7-Dihydroxy-naphthalin, 2,3-Dihydroxy-naphthalin, 2,7-Dihydroxy-naphthalin, 2-Methyl-1-naphthol-acetat, 1,3-Dihydroxy-benzol, 1-Chlor-2,4-dihydroxy-benzol, 2-Chlor-1,3-dihydroxy-benzol, 1,2-Dichlor-3,5-dihydroxy-4-methyl-benzol, 1,5-Dichlor-2,4-dihydroxybenzol, 1,3-Dihydroxy-2-methyl-benzol, 3,4-Methylendioxy-phenol, 3,4-Methylendioxy-anilin, 5-[(2-Hydroxyethyl)amino]-1,3-benzodioxol, 6-Brom-1-hydroxy-3,4-methylendioxy-benzol, 3,4-Diamino-benzoesäure, 3,4-Dihydro-6-hydroxy-1,4(2H)-benzoxazin, 6-Amino-3,4-dihydro-1,4(2H)-benzoxazin, 3-Methyl-1-phenyl-5-pyrazolon, 5,6-Dihydroxy-indol, 5,6-Dihydroxy-indolin, 5-Hydroxy-indol, 6-Hydroxy-indol, 7-Hydroxy-indol und 2,3-Indolindion enthält.

9. Mittel nach einem der Ansprüche 4 bis 8 , **dadurch gekennzeichnet, dass** die Entwicklersubstanzen und Kupplersubstanzen, bezogen auf die Gesamtmenge des Färbemittels, jeweils in einer Gesamtmenge von 0,005 bis 20 Gewichtsprozent enthalten sind.

10. Mittel nach einem der Ansprüche 4 bis 9, **dadurch gekennzeichnet, dass** es zusätzlich mindestens einen direktziehenden Farbstoff enthält.

11. Mittel nach einem der Ansprüche 4 bis 10, **dadurch gekennzeichnet, dass** es einen pH-Wert von 6,5 bis 11,5 aufweist.

12. Mittel nach einem der Ansprüche 4 bis 11, **dadurch gekennzeichnet, dass** es ein Haarfärbemittel ist.

## Claims

1. 1,3-Dihydroxy-4-heteroarylbenzene derivative of the general formula (I) or physiologically compatible, water-soluble salts thereof, 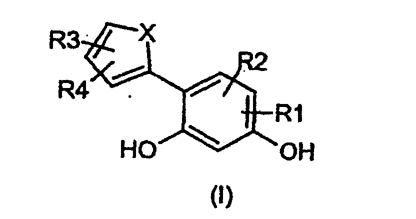 in which
**X** is oxygen, sulphur or N-R5;
**R1** and **R2** may be identical or different and, independently of one another, are hydrogen, a halogen atom, a hydroxy group, a C₁-C₄-alkoxy group, a C₁-C₄-hydroxyalkoxy group, a C₁-C₆-alkyl group, a C₁-C₄-alkyl thioether group, a trifluoromethane group, a -Si(CH₃)₃ group, a C₁-C₄ hydroxyalkyl group or a C₂-C₄-dihydroxyalkyl group;
**R3** and **R4** may be identical or different and, independently of one another, are hydrogen, a hydroxy group, a halogen atom, a cyano group, a C₁-C₄-alkoxy group, a C₁-C₆-alkyl group, a C₁-C₄-alkyl thioether group, a mercapto group, a nitro group, an amino group, a C₁-C₄-alkylamino group, a di(C₁-C₄)alkylamino group, a -C(O)H group, a -C(O)CH₃ group, a -C(O)CF₃ group, a -Si(CH₃)₃ group, a C₁-C₄-hydroxyalkyl group, a C₂-C₄-dihydroxyalkyl group; and
**R5** is hydrogen, a C₁-C₆-alkyl group, a C₂-C₄-hydroxyalkyl group, a phenyl group or an acetyl group.

2. 1,3-Dihydroxy-4-heteroarylbenzene derivative according to Claim 1, **characterized in that** it is chosen from compounds of the formula (I) in which (i) one or more of the radical groups **R1, R2, R3** and **R4** are hydrogen and **X** is sulphur or oxygen or (ii) **R1** and **R2** are hydrogen at the same time and **X** is sulphur.

3. 1,3-Dihydroxy-4-heteroarylbenzene derivative of the formula (I) according to Claim 1 or 2, **characterized in that** it is chosen from the group consisting of 1,3-dihydroxy-4-(thiophen-2-yl)benzene, 1,3-dihydroxy-4-(5-nitrothiophen-2-yl)benzene, 1,3-dihydroxy-4-(3-methylthiophen-2-yl)benzene and 1,3-dihydroxy-4-(furan-2-yl)benzene and physiologically compatible salts thereof.

4. Agent for the oxidative colouring of keratin fibres based on a developer substance-coupler substance combination, **characterized in that** it comprises at least one 1,3-dihydroxy-4-heteroarylbenzene derivative of the formula (I) according to one of Claims 1 to 3.

5. Agent according to Claim 4, **characterized in that** the compound of the formula (I) is chosen from the group consisting of 1,3-dihydroxy-4-(thiophen-2-yl)-benzene, 1,3-dihydroxy-4-(5-nitrothiophen-2-yl)benzene, 1,3-dihydroxy-4-(3-methylthiophen-2-yl)benzene and 1,3-dihydroxy-4-(furan-2-yl)benzene, and physiologically compatible salts thereof.

6. Agent according to Claim 4 or 5, **characterized in that** the 1,3-dihydroxy-4-heteroarylbenzene of the formula (I) is present in an amount of from 0.005 to 20 percent by weight.

7. Agent according to one of Claims 4 to 6, **characterized in that** the developer substance is chosen from the group consisting of 1,4-diaminobenzene, 1,4-diamino-2-methylbenzene, 1,4-diamino-2,6-dimethylbenzene, 1,4-diamino-3,5-diethylbenzene, 1,4-diamino-2,5-dimethylbenzene, 1,4-diamino-2,3-dimethylbenzene, 2-chloro-1,4-diaminobenzene, 1,4-diamino-2-(thiophen-2-yl)benzene, 1,4-diamino-2-(thiophen-3-yl)benzene, 1,4-diamino-2-(pyridin-3-yl)benzene, 2,5-diaminobiphenyl, 1,4-diamino-2-methoxymethylbenzene, 1,4-diamino-2-aminomethylbenzene, 1,4-diamino-2-hydroxymethylbenzene, 1,4-diamino-2-(2-hydroxyethoxy)benzene, 2-(2-(acetylamino)ethoxy)-1,4-diaminobenzene, 4-phenylaminoaniline, 4-dimethylaminoaniline, 4-diethylaminoaniline, 4-dipropylaminoaniline, 4-[ethyl(2-hydroxyethyl)amino]aniline, 4-[di(2-hydroxyethyl)amino]-aniline, 4-[di(2-hydroxyethyl)amino]-2-methylaniline, 4-[(2-methoxyethyl)amino]aniline, 4-[(3-hydroxypropyl)-amino]aniline, 4-[(2,3-dihydroxypropyl)amino]aniline, 1,4-diamino-2-(1-hydroxyethyl)benzene, 1,4-diamino-2-(2-hydroxyethyl)benzene, 1,4-diamino-2-(1-methylethyl)benzene, 1,3-bis[(4-aminophenyl)(2-hydroxyethyl)amino]-2-propanol, 1,4-bis[(4-aminophenyl)amino]-butane, 1,8-bis(2,5-diaminophenoxy)-3,6-dioxaoctane, 4-aminophenol, 4-amino-3-methylphenol, 4-amino-3-(hydroxymethyl)phenol, 4-amino-3-fluorophenol, 4-methylaminophenol, 4-amino-2-(aminomethyl)phenol, 4-amino-2-(hydroxymethyl)phenol, 4-amino-2-fluorophenol, 4-amino-2-[(2-hydroxyethyl)amino]methylphenol, 4-amino-2-methylphenol, 4-amino-2-(methoxymethyl)-phenol, 4-amino-2-(2-hydroxyethyl)phenol, 5-aminosalicylic acid, 2,5-diaminopyridine, 2,4,5,6-tetraaminopyrimidine, 2,5,6-triamino-4-(1H)pyrimidone, 4,5-diamino-1-(2-hydroxyethyl)-1H-pyrazole, 4,5-diamino-1-(1-methylethyl)-1H-pyrazole, 4,5-diamino-1-[(4-methylphenyl)methyl]-1H-pyrazole, 2-[(4-chlorophenyl)methyl]-4,5-diamino-1H-pyrazole, 4,5-diamino-1-methyl-1H-pyrazole, 2-aminophenol, 2-amino-6-methylphenol, 2-amino-5-methylphenol and 1,2,4-trihydroxybenzene.

8. Agent according to one of Claims 4 to 7, **characterized in that**, in addition to the compounds of the formula (I), it also comprises further known coupler substances from the group consisting of N-(3-dimethylaminophenyl)urea, 2,6-diaminopyridine, 2-amino-4-[(2-hydroxyethyl)amino]anisole, 2,4-diamino-1-fluoro-5-methylbenzene, 2,4-diamino-1-methoxy-5-methylbenzene, 2,4-diamino-1-ethoxy-5-methylbenzene, 2,4-diamino-1-(2-hydroxyethoxy)-5-methylbenzene, 2,4-di[(2-hydroxyethyl)amino]-1,5-dimethoxybenzene, 2,3-diamino-6-methoxypyridine, 3-amino-6-methoxy-2-(methylamino)pyridine, 2,6-diamino-3,5-dimethoxypyridine, 3,5-diamino-2,6-dimethoxypyridine, 1,3-diaminobenzene, 2,4-diamino-1-(2-hydroxyethoxy)benzene, 1,3-diamino-4-(2,3-dihydroxypropoxy)-benzene, 1,3-diamino-4-(3-hydroxypropoxy)benzene, 1,3-diamino-4-(2-methoxyethoxy)benzene, 2,4-diamino-1,5-di(2-hydroxyethoxy)benzene, 1-(2-aminoethoxy)-2,4-diaminobenzene, 2-amino-1-(2-hydroxyethoxy)-4-methylaminobenzene, 2,4-diaminophenoxyacetic acid, 3-[di(2-hydroxyethyl)amino]aniline, 4-amino-2-di[(2-hydroxyethyl)amino]-1-ethoxybenzene, 5-methyl-2-(1-methylethyl)phenol, 3-[(2-hydroxyethyl)amino]aniline, 3-[(2-aminoethyl)amino]aniline, 1,3-di(2,4-diaminophenoxy)-propane, di(2,4-diaminophenoxy)methane, 1,3-diamino-2,4-dimethoxybenzene, 2,6-bis(2-hydroxyethyl)aminotoluene, 4-hydroxyindole, 3-dimethylaminophenol, 3-diethylaminophenol, 5-amino-2-methylphenol, 5-amino-4-fluoro-2-methylphenol, 5-amino-4-methoxy-2-methylphenol, 5-amino-4-ethoxy-2-methylphenol, 3-amino-2,4-dichlorophenol, 5-amino-2,4-dichlorophenol, 3-amino-2-methylphenol, 3-amino-2-chloro-6-methylphenol, 3-aminophenol, 2-[(3-hydroxyphenyl)amino]acetamide, 5-[(2-hydroxyethyl)amino]-4-methoxy-2-methylphenol, 5-[(2-hydroxyethyl)amino]-2-methylphenol, 3-[(2-hydroxyethyl)amino]phenol, 3-[(2-methoxyethyl)amino]phenol 5-amino-2-ethylphenol, 5-amino-2-methoxyphenol, 2-(4-amino-2-hydroxyphenoxy)ethanol, 5-[(3-hydroxypropyl)-amino]-2-methylphenol, 3-[(2,3-dihydroxypropyl)amino]-2-methylphenol, 3-[(2-hydroxyethyl)amino]-2-methylphenol, 2-amino-3-hydroxypyridine, 2,6-dihydroxy-3,4-dimethylpyridine, 5-amino-4-chloro-2-methylphenol, 1-naphthol, 2-methyl-1-naphthol, 1,5-dihydroxynaphthalene, 1,7-dihydroxynaphthalene, 2,3-dihydroxynaphthalene, 2,7-dihydroxynaphthalene, 2-methyl-1-naphthol acetate, 1,3-dihydroxybenzene, 1-chloro-2,4-dihydroxybenzene, 2-chloro-1,3-dihydroxybenzene, 1,2-dichloro-3,5-dihydroxy-4-methylbenzene, 1,5-dichloro-2,4-dihydroxybenzene, 1,3-dihydroxy-2-methylbenzene, 3,4-methylenedioxyphenol, 3,4-methylenedioxyaniline, 5-[(2-hydroxyethyl)amino)-1,3-benzodioxole, 6-bromo-1-hydroxy-3,4-methylenedioxybenzene, 3,4-diaminobenzoic acid, 3,4-dihydro-6-hydroxy-1,4(2H)benzoxazine, 6-amino-3,4-dihydro-1,4(2H)benzoxazine, 3-methyl-1-phenyl-5-pyrazolone, 5,6-dihydroxyindole, 5,6-dihydroxyindoline, 5-hydroxyindole, 6-hydroxyindole, 7-hydroxyindole and 2,3-indolinedione.

9. Agent according to one of Claims 4 to 8, **characterized in that** the developer substances and coupler substances, based on the total amount of the colorant, are in each case present in a total amount of from 0.005 to 20 percent by weight.

10. Agent according to one of Claims 4 to 9, **characterized in that** it additionally comprises at least one direct dye.

11. Agent according to one of Claims 4 to 10, **characterized in that** it has a pH of from 6.5 to 11.5.

12. Agent according to one of Claims 4 to 11, **characterized in that** it is a hair colorant.

## Revendications

1. Dérivé de 1,3-dihydroxy-4-hétéroarylbenzène de formule générale (I) ou sels hydrosolubles physiologiquement acceptables d'un tel dérivé, 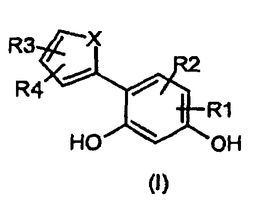 formule dans laquelle
**X** est un atome d'oxygène, un atome de soufre ou N-R5 ;
**R1** et **R2** peuvent être identiques ou différents et représentent, indépendamment l'un de l'autre, un atome d'hydrogène, un atome d'halogène, un groupe hydroxy, un groupe alcoxy en C₁-C₄, un groupe hydroxyalcoxy en C₁-C₄, un groupe alkyle en C₁-C₆, un groupe alkyl(C₁-C₄)thioéther, un groupe trifluorométhane, un groupe -Si(CH₃)₃, un groupe hydroxyalkyle en C₁-C₄ ou un groupe dihydroxyalkyle en C₂-C₄ ;
**R3** et **R4** peuvent être identiques ou différents et représentent, indépendamment l'un de l'autre, un atome d'hydrogène, un groupe hydroxy, un atome d'halogène, un groupe cyano, un groupe alcoxy en C₁-C₄, un groupe alkyle en C₁-C₆, un groupe alkyl-(C₁-C₄)thioéther, un groupe mercapto, un groupe nitro, un groupe amino, un groupe alkyl(C₁-C₄)amino, un groupe dialkyl(C₁-C₄)amino, un groupe -C(O)H, un groupe -C(O)CH₃, un groupe -C(O)CF₃, un groupe -Si (CH₃)₃, un groupe hydroxyalkyle en C₁-C₄, un groupe dihydroxyalkyle en C₂-C₄ ; et
**R5** est un atome d'hydrogène, un groupe alkyle en C₁-C₆, un groupe hydroxyalkyle en C₂-C₄, un groupe phényle ou un groupe acétyle.

2. Dérivé de 1,3-dihydroxy-4-hétéroarylbenzène selon la revendication 1, **caractérisé en ce qu'**il est choisi parmi des composés de formule (I) dans lesquels (i) un ou plusieurs des groupes **R1, R2, R3** et **R4** représentent un atome d'hydrogène et **X** est un atome de soufre ou d'oxygène, ou (ii) **R1** et **R2** représentent simultanément un atome d'hydrogène et **X** est un atome de soufre.

3. Dérivé de 1,3-dihydroxy-4-hétéroarylbenzène de formule (I) selon la revendication 1 ou 2, **caractérisé en ce qu'**il est choisi dans le groupe constitué par le 1,3-dihydroxy-4-(thiophén-2-yl)benzène, le 1,3-dihydroxy-4-(5-nitrothiophén-2-yl)benzène, le 1,3-dihydroxy-4-(3-méthylthiophén-2-yl)benzène et le 1,3-dihydroxy-4-(furann-2-yl)benzène ainsi que leurs sels physiologiquement acceptables.

4. Composition pour la teinture par oxydation de fibres de kératine à base d'une association développeur-coupleur, **caractérisée en ce qu'**elle contient au moins un dérivé de 1,3-dihydroxy-4-hétéroarylbenzène de formule (I) selon l'une quelconque des revendications 1 à 3.

5. Composition selon la revendication 4 **caractérisée en ce que** le composé de formule (I) est choisi dans le groupe constitué par le 1,3-dihydroxy-4-(thiophén-2-yl)benzène, le 1,3-dihydroxy-4-(5-nitrothiophén-2-yl)benzène, le 1,3-dihydroxy-4-(3-méthylthiophén-2-yl)benzène et le 1,3-dihydroxy-4-(furann-2-yl)benzène ainsi que leurs sels physiologiquement acceptables.

6. Composition selon la revendication 4 ou 5, **caractérisée en ce que** le 1,3-dihydroxy-4-hétéroarylbenzène de formule (I) est contenu en une quantité de 0,005 à 20 % en poids.

7. Composition selon l'une quelconque des revendications 4 à 6, **caractérisée en ce que** le développeur est choisi dans le groupe constitué par le 1,4-diaminobenzène, le 1,4-diamino-2-méthylbenzène, le 1,4-diamino-2,6-diméthylbenzène, le 1,4-diamino-3,5-diéthylbenzène, le 1,4-diamino-2,5-diméthylbenzène, le 1,4-diamino-2,3-diméthylbenzène, le 2-chloro-1,4-diaminobenzène, le 1,4-diamino-2-(thiophén-2-yl)benzène, le 1,4-diamino-2-(thiophén-3-yl)benzène, le 1,4-diamino-2-(pyridin-3-yl)benzène, le 2,5-diaminobiphényle, le 1,4-diamino-2-méthoxyméthylbenzène, le 1,4-diamino-2-aminométhylbenzène, le 1,4-diamino-2-hydroxyméthylbenzène, le 1,4-diamino-2-(2-hydroxyéthoxy)-benzène, le 2-(2-(acétylamino)éthoxy)-1,4-diaminobenzène, la 4-phénylamino-aniline, 4-diméthylamino-aniline, la 4-diéthylamino-aniline, la 4-dipropylamino-aniline, la 4-[éthyl-(2-hydroxyéthyl)amino]aniline, la 4-[di(2-hydroxyéthyl)-amino]aniline, la 4-[di(2-hydroxyéthyl)amino]-2-méthylaniline, la 4-[(2-méthoxyéthyl)amino]-aniline, la 4-[(3-hydroxypropyl)amino]aniline, la 4-[(2,3-dihydroxypropyl)amino]aniline, le 1,4-diamino-2-(1-hydroxyéthyl)benzène, le 1,4-diamino-2-(2-hydroxyéthyl)benzène, le 1,4-diamino-2-(1-méthyléthyl)benzène, le 1,3-bis[(4-aminophényl)(2-hydroxyéthyl)amino]-2-propanol, le 1,4-bis[(4-aminophényl)amino]butane, le 1,8-bis(2,5-diaminophénoxy)-3,6-dioxaoctane, le 4-aminophénol, le 4-amino-3-méthylphénol, le 4-amino-3-(hydroxyméthyl)phénol, le 4-amino-3-fluorophénol, le 4-méthylaminophénol, le 4-amino-2-(aminométhyl)-phénol, le 4-amino-2-(hydroxyméthyl)phénol, le 4-amino-2-fluorophénol, le 4-amino-2-[(2-hydroxyéthyl)amino]méthylphénol, le 4-amino-2-méthylphénol, le 4-amino-2-(méthoxyméthyl)phénol, le 4-amino-2-(2-hydroxyéthyl)phénol, l'acide 5-aminosalicylique, la 2,5-diaminopyridine, la 2,4,5,6-tétraaminopyrimidine, la 2,5,6-triamino-4(1H)-pyrimidone, le 4,5-diamino-1-(2-hydroxyéthyl)-1H-pyrazole, le 4,5-diamino-1-(1-méthyléthyl)-1H-pyrazole, le 4,5-diamino-1-[(4-méthylphényl)méthyl]-1H-pyrazole, le 1-[(4-chlorophényl)méthyl]-4,5-diamino-1H-pyrazole, le 4,5-diamino-1-méthyl-1H-pyrazole, le 2-aminophénol, le 2-amino-6-méthylphénol, le 2-amino-5-méthylphénol et le 1,2,4-trihydroxybenzène.

8. Composition selon l'une quelconque des revendications 4 à 7, **caractérisée en ce qu'**en plus des composés de formule (I), elle contient encore d'autres coupleurs connus choisis dans le groupe constitué par la N-(3-diméthylaminophényl)urée, la 2,6-diaminopyridine, le 2-amino-4-[(2-hydroxyéthyl)amino]anisole, le 2,4-diamino-1-fluoro-5-méthylbenzène, le 2,4-diamino-1-méthoxy-5-méthylbenzène, le 2,4-diamino-1-éthoxy-5-méthylbenzène, le 2,4-diamino-1-(2-hydroxyéthoxy)-5-méthylbenzène, le 2,4-di[(2-hydroxyéthyl)amino]-1,5-diméthoxybenzène, la 2,3-diamino-6-méthoxypyridine, la 3-amino-6-méthoxy-2-(méthylamino)-pyridine, la 2,6-diamino-3,5-diméthoxypyridine, la 3,5-diamino-2,6-diméthoxypyridine, le 1,3-diaminobenzène, le 2,4-diamino-1-(2-hydroxyéthoxy)-benzène, le 1,3-diamino-4-(2,3-dihydroxypropoxy)-benzène, le 2,3-diamino-4-(3-hydroxypropoxy)-benzène, le 1,3-diamino-4-(2-méthoxyéthoxy)-benzène, le 2,4-diamino-1,5-di(2-hydroxyéthoxy)-benzène, le 1-(2-aminoéthoxy)-2,4-diaminobenzène, le 2-amino-1-(2-hydroxyéthoxy)-4-méthylaminobenzène, l'acide 2,4-diaminophénoxyacétique, la 3-[di(2-hydroxyéthyl)amino]aniline, le 4-amino-2-di[(2-hydroxyéthyl)amino]-1-éthoxybenzène, le 5-méthyl-2-(1-méthyléthyl)phénol, la 3-[(2-hydroxyéthyl)amino]aniline, la 3-[(2-aminoéthyl)-amino]aniline, le 1,3-di(2,4-diaminophénoxy)-propane, le di(2,4-diaminophénoxy)méthane, le 1,3-diamino-2,4-diméthoxybenzène, le 2,6-bis(2-hydroxyéthyl)aminotoluène, le 4-hydroxy-indole, le 3-diméthylaminophénol, le 3-diéthylaminophénol, le 5-amino-2-méthylphénol, le 5-amino-4-fluoro-2-méthylphénol, le 5-amino-4-méthoxy-2-méthylphénol, le 5-amino-4-éthoxy-2-méthylphénol, le 3-amino-2,4-dichlorophénol, le 5-amino-2,4-dichlorophénol, le 3-amino-2-méthylphénol, le 3-amino-2-chloro-6-méthylphénol, le 3-aminophénol, le 2-[(3-hydroxyphényl)amino]acétamide, le 5-[(2-hydroxyéthyl)-amino]-4-méthoxy-2-méthylphénol, le 5-[(2-hydroxyéthyl)amino]-2-méthylphénol, le 3-[(2-hydroxyéthyl)amino]phénol, le 3-[(2-méthoxyéthyl)amino]-phénol, le 5-amino-2-éthylphénol, le 5-amino-2-méthoxyphénol, le 2-(4-amino-2-hydroxyphénoxy)-éthanol, le 5-[(3-hydroxypropyl)amino]-2-méthylphénol, le 3-[(2,3-dihydroxypropyl)amino]-2-méthylphénol, le 3-[(2-hydroxyéthyl)amino]-2-méthylphénol, la 2-amino-3-hydroxypyridine, le 2,6-dihydroxy-3,4-diméthylpyridine, le 5-amino-4-chloro-2-méthylphénol, le 1-naphtol, le 2-méthyl-1-naphthol, le 1,5-dihydroxynaphthalène, le 1,7-dihydroxynaphthalène, le 2,3-dihydroxynaphthalène, le 2,7-dihydroxynaphthalène, l'acétate de 2-méthyl-1-naphtol, le 1,3-dihydroxybenzène, le 1-chloro-2,4-dihydroxybenzène, le 2-chloro-1,3-dihydroxybenzène, le 1,2-dichloro-3,5-dihydroxy-4-méthylbenzène, le 1,5-dichloro-2,4-dihydroxybenzène, le 1,3-dihydroxy-2-méthylbenzène, le 3,4-méthylènedioxyphénol, la 3,4-méthylènedioxyaniline, le 5-[(2-hydroxyéthyl)amino]-1,3-benzodioxole, le 6-bromo-1-hydroxy-3,4-méthylènedioxybenzène, l'acide 3,4-diaminobenzoïque, la 3,4-dihydro-6-hydroxy-1,4(2H)-benzoxazine, la 6-amino-3,4-dihydro-1,4(2H)-benzoxazine, la 3-méthyl-1-phényl-5-pyrazolone, le 5,6-dihydroxyindole, la 5,6-dihydroxy-indoline, le 5-hydroxyindole, le 6-hydroxy-indole, le 7-hydroxy-indole et la 2,3-indolinedione.

9. Composition selon l'une quelconque des revendications 4 à 8, **caractérisée en ce que** les développeurs et les coupleurs sont contenus, respectivement, en une quantité totale de 0,005 à 20 % en poids, par rapport à la quantité totale de la composition de teinture.

10. Composition selon l'une quelconque des revendications 4 à 9, **caractérisée en ce qu'**en outre elle contient au moins un colorant direct.

11. Composition selon l'une quelconque des revendications 4 à 10, **caractérisée en ce qu'**elle présente un pH de 6,5 à 11,5.

12. Composition selon l'une quelconque des revendications 4 à 11, **caractérisée en ce qu'**il s'agit d'une composition de teinture pour cheveux.
